# EUROPEAN PATENT SPECIFICATION

(11) **EP 1 906 881 B1**
(45) Date of publication and mention of the grant of the patent: **15.08.2012**
(21) Application number: 05747641.8
(22) Date of filing: 11.05.2005
(51) Int. Cl.: A61F 2/16

(54) **INTRAOCULAR MULTIFOCAL LENS**
MULTIFOKALE INTRAOKULARLINSE
LENTILLE MULTIFOCALE INTRAOCULAIRE

(43) Date of publication of application: 09.04.2008
(73) Proprietor: McDonald, Marguerite, B., New Orleans, LA 70115 (US)
(72) Inventor: McDonald, Marguerite, B., New Orleans, LA 70115 (US)
(74) Representative: Haslam, Simon David
(86) International application number: PCT/US2005/016537
(87) International publication number: WO 2006/124016

(56) References cited:
- US-A- 4 512 040
- US-A- 5 522 891
- US-A1- 2002 101 564
- US-A1- 2003 083 744

## Description

### TECHNICAL FIELD OF THE INVENTION

This invention relates to a intraocular lenses (IOLs), specifically, to IOLs that may have a plurality of refractive indices.

### BACKGROUND OF THE INVENTION

The human eye includes an anterior chamber between the cornea and iris, a posterior chamber, defined by a capsular bag, containing a crystalline lens enclosed by a clear capsule, a vitreous chamber behind the lens containing the vitreous humor, and a retina at the rear of this chamber. The human eye has a natural accommodation ability. The constriction or contraction and relaxation of the ciliary muscle provides the eye with near and distant vision, respectively. This ciliary muscle action shapes the natural crystalline lens to the appropriate optical configuration for focusing light rays entering the eye on the retina. The removal of the natural lens leaves the eye with no means to focus at different distances and necessitates the use of bifocal lenses for near and far work.

Many different IOL designs have been developed over the years and proven successful in phakic and aphakic eyes. IOL implants have been used for years in aphakic eyes as replacements for diseased natural crystalline lenses that have been surgically removed from the eyes. IOLs for aphakic eyes are now implanted after cataract surgery in over 98 % of the cases. Visual acuity deficiencies remaining after cataract surgery such as myopia (nearsightedness), hyperopia (farsightedness), and presbyopia (age-related farsightedness) are typically corrected with eyeglasses or contact lenses, though the accuracy of IOL power calculation has decreased the need for full-time postoperative spectacle use. The use of surgically implanted phakic IOLs as a permanent form of refractive correction has been gaining in popularity as well, though they are still considered investigational.

IOLs are generally of a fixed focal length. Fused bifocal and aspheric lenses have two or more optical focal planes that are engaged by changing the relationship of the light path or the observer to the lens. All such lenses require movement of either the lens (not possible with a standard IOL), light path, or observer to engage a different focal plane and can therefore be difficult to use. They also have an adverse effect on contrast sensitivity and can cause glare, halos, double vision (polyopia), and other optical aberration all of which compromises visual quality. A multifocal intraocular lens comprising a lens body having elliptical anterior and posterior surfaces, which requires a shift of the lens when the optical axis is placed at an incline is known from US-A-5 522 891.

Accordingly, there is a need for a multifocal intraocular lens having a variable power of refraction that does not require deliberate engagement of different focal planes for near and far vision.

### SUMMARY OF THE INVENTION

In accordance with the present invention, a multifocal intraocular lens provides greater or lesser refraction in relation to the position of the head and eyes of a user. A multifocal intraocular lens body for insertion into a fluid- or gel-filled enucleated natural lens capsule of an eye is provided wherein the lens body encompasses the optical axis of the eye and provides different greater or lesser refraction depending upon the position of the eye. In a second embodiment, the lens body can be used with an artificial lens capsule implanted within an eye.

### BRIEF DESCRIPTION OF THE DRAWINGS

For a more complete understanding of the present invention and for further advantages thereof, reference is now made to the following Description of the Preferred Embodiments taken in conjunction with the accompanying Drawings in which:
FIG. 1 is a sectional view of a human eye illustrating the basic components of the eye;
FIG. 2 is a sectional view of a human eye illustrating the present intraocular lens body inserted within the artificial capsule situated within an eye;
FIG. 3 is a side-elevated view of the present intraocular lens body and artificial capsule shown in FIG. 2.
FIG. 4 is a plan view of the present intraocular lens body shown in FIG. 2; and
FIG. 5 is a sectional view of a human eye illustrating the present intraocular lens body inserted within the natural lens capsule of an eye.
FIG. 6 is a sectional view of the present intraocular lens body through the line 6-6 of FIG. 3.
FIG. 7 is a sectional view of the present intraocular lens body through the line 7-7 of FIG. 3.

### DETAILED DESCRIPTION OF THE INVENTION

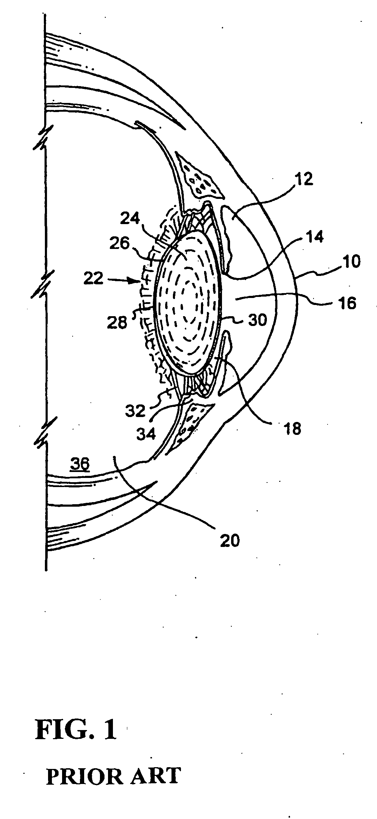

Turning now to these drawings and first to FIG. 1 for reference in the following description, the human eye comprises from front to rear an outer transparent cornea 10, an anterior chamber 12, an iris 14 containing a pupil 16, a posterior chamber 18, and a vitreous cavity 20. A natural lens 22 comprises a crystalline lens structure 24 contained within an optically clear bag called a lens capsule 26 having a posterior capsule 28 and an anterior capsule 30. The lens is peripherally joined by zonules 32 to the surrounding ciliary muscles 34. The vitreous cavity 20 contains vitreous 36.

The present invention is particularly useful in correcting the eye disorder known as a cataract which is characterized by progressive opacification of the natural lens 22 and resultant progressive attenuation of the light rays reaching the retina. Simply stated, this condition is corrected by removing the cataract, that is the cataractous (i.e. opaque) nucleus and cortex of the natural lens, and implanting an artificial intraocular lens in the eye. Cataract removal may be accomplished by intracapsular extraction (removal of the entire lens 22) or by extracapsular extraction (removal of the cataractous nucleus and cortex through the anterior side of the lens capsule 26) utilizing either nucleus expression through a relatively large opening in the eye or phacoemulsification through a relatively small opening in the eye.

The anterior capsule opening required for extracapsular extraction may be provided by (a) removing most of the anterior capsule 30 except small peripheral remnants of the anterior capsule, (b) tearing the anterior capsule to allow the human lens nucleus to be expressed, or (c) continuous tear circular capsulotomy, i.e. capsulorhexis. The essentially empty lens capsule 26 remaining after removal of the cataract is referred to as a capsular bag. Removal of the nucleus and cortex of the natural lens from the bag creates a space immediately behind the iris 14, between the latter and the posterior capsule 28 of the bag, which is approximately 10 mm in diameter and 5 mm front to back.

It is possible to implant an intraocular lens in any one or more of the eye chambers, i.e. anterior chamber 12, posterior chamber 18, capsular bag 26, or vitreous cavity 20. Intraocular implants according to the present invention may be placed in any one of these eye chambers or cavities. However, the invention is particularly concerned with an intraocular lens body for placement in a natural capsular bag having an anterior capsulotomy or in the anterior or posterior chambers, or in an artificial capsular bag and will be described primarily in these contexts.

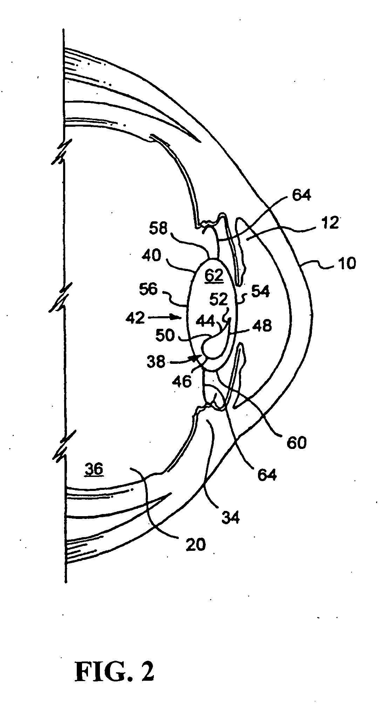

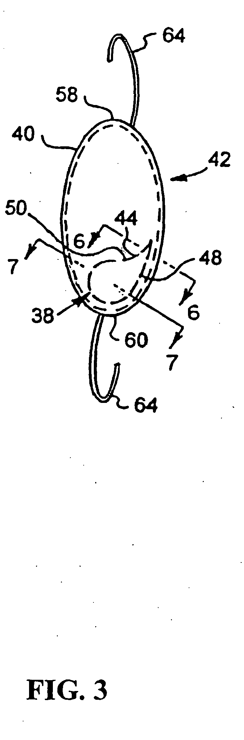

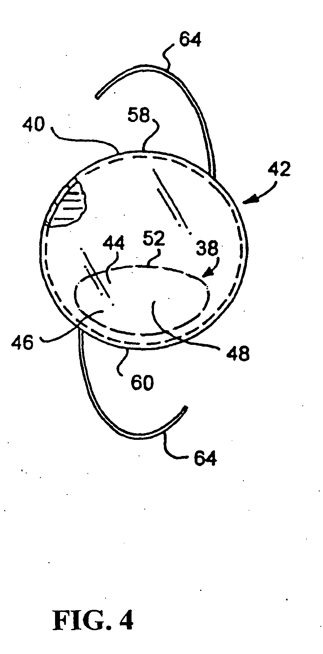

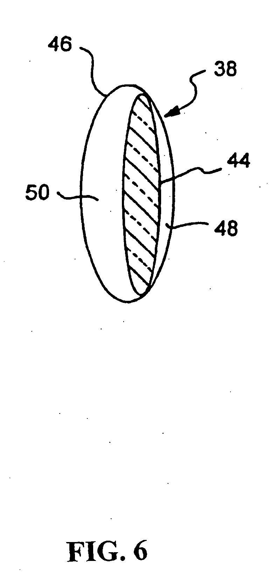

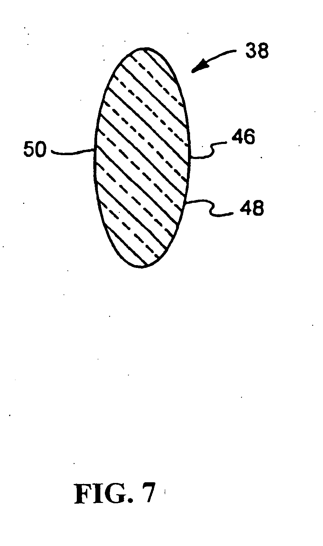

Referring now to FIGS. 2-4, in accordance with the present invention, there is illustrated multifocal intraocular lens body, generally identified by the numeral 38, positioned within an artificial capsular bag 40 to form a multifocal intraocular lens system, generally identified by the numeral 42, situated within a human eye from which the natural lens 22 has been removed (i.e. an aphakic eye). Multifocal intraocular lens body 38 has an upper portion 44, a lower portion 46, anterior surface 48, and a posterior surface 50. The following description of the lens body is as viewed from the medial-lateral axis. Anterior surface 48 and posterior surface 50 of lower portion 46 taper upwardly to form upper portion 44 to provide a tapering periphery 52. Anterior surface 48 and posterior surface 50 of upper portion 44 of lens body 38 each have at least one radius of curvature having its respective center positioned posteriorly the posterior surface 50 of lens body 38. An aspheric multifocal intraocular lens body is contemplated in one embodiment of the present lens body. At least one radius of curvature of posterior surface 50 of upper portion 44 of the, lens body is preferably shorter than the radius of curvature of that along anterior surface 48 so as to form a comma-shaped lens body or any multifocal shape, preferably aspheric. Multiple radii of curvature are also contemplated as well as possibly a lens made of a material that increases in its index of refraction along the lens axes. As seen FIGS. 4, 6 and 7, the lens body is approximately elliptical when viewed anteriorly or posteriorly. The multifocal intraocular lens body may comprise any material suitable as a lens body and having a high index of refraction. Such material is preferably translucent and colorless, such as silicone, gel, or acrylic, and specifically may include polymethylmethacrylate (PMMA). However, such material may have a color for aesthetic or physiological purposes, e.g. to filter unwanted wavelengths such as those in the ultraviolet spectrum.

Multifocal intraocular lens body 38 is positioned within lens capsule 40. Lens capsule 40 has anterior surface 54, posterior surface 56, upper edge 58, and lower edge 60. Lens capsule 40 is approximately circular in shape and adapted to fit in the anterior or posterior chambers of an eye. Anterior surface 54 and posterior surface 56 completely encompass the optical axis formed by pupil 16. The distance between anterior surface 48 and posterior surface 56 of lens capsule 40 defines a thickness that corresponds to a first axis that extends generally perpendicularly between the surfaces. A second axis that is generally perpendicular to said first axis is also formed. The thickness of the lens capsule along the first axis is smaller than its width along its second axis. Lens capsule 40 can be made from a translucent and colorless pliable or substantially pliable material such as silicone or similar material, or other materials as would be known to one of skill in the art.

The multifocal intraocular lens body 38 is positioned within the lens capsule 40 so that lower portion 46 of lens body 38 lies adjacent to lower edge 60 of the lens capsule and anterior surface 48 of the lens body lies adjacent to anterior surface 54 of lens capsule 40. Tapering periphery 52 is the feature of lens body 38 that lies nearest to the approximate center of the lens capsule when the short axis of the lens capsule is parallel to the ground or as when the user is looking straight ahead. The approximate center of the lens capsule encompasses the optical axis of the eye. Multifocal intraocular lens body 38 provides only minimal additional refraction or no refraction consummate with the minimal or no additional refraction or required when looking at infinity, i.e. at the horizon. When anterior surface 54 of lens capsule 40 is tilted downwardly, lens body 38 moves towards upper edge 58 of the lens capsule and begins to "float" or slide more and more over the visual axis (i.e. the line of sight). After implantation, and when the user requires more focusing power, i.e. refraction, such as when reading, lens body 38 similarly slides upwards into the optical axis as the user tilts their head downwardly causing anterior surface 54 of lens capsule 40 to also move downwardly. By doing so, the lower portion of the lens body which has a higher add component, moves into the optical axis and provides greater correction at near. Movement of the multifocal intraocular lens body 38 within artificial capsule bag 40 is slowed by the presence of translucent fluid 62 contained within bag 40. Translucent fluid 62 may be a liquid, gel, or sol, is preferably visco-elastic, and can be silicone or a related material or a combination of materials. However, any translucent substance capable of slowing movement of the multifocal intraocular lens body 38 within the artificial capsule bag 40 consistent with the practice of this invention is suitable.

In another embodiment of the present invention, attached to the outside edge of artificial capsule bag 40 is securer 64. Securer 64 secures artificial capsule bag 40 containing multifocal intraocular lens body 38 within the eye. Securer 64 are preferably positioned so as to extend from upper edge 58 and lower edge 60 of artificial capsule bag 40 and hold the artificial capsule bag 40 in place within either the posterior chamber, the capsular bag or remnants thereof, or anterior chamber, or a combination thereof. Haptics 64, as shown in FIGS. 2-4, are commonly used in the art. Any haptic shape, configuration, or number may be utilized in accordance with the teachings of the present invention. While haptics are satisfactory for the present invention, any other suitable securer is acceptable if a securer is desired.

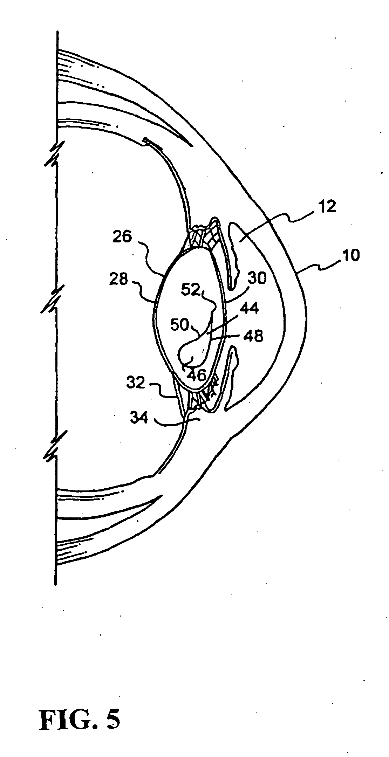

Referring now to FIG. 5, there is illustrated multifocal intraocular lens body 38 according to another embodiment of the present invention. Multifocal intraocular lens body 38 is placed within capsular bag 26 of the human phakic eye from which the nucleus of the natural lens has been removed, i.e. the lens is enucleated. The capsular bag 26 has an anterior capsulotomy (not shown) through which the lens body 38 is inserted into the bag 26. In this embodiment the natural lens capsule 26 contains the lens body 38 rather than the artificial capsule bag 40 as described above. The anterior capsulotomy, probably small in size, may be plugged with a natural or synthetic material after the lens body is inserted and the capsular bag filled with a translucent substance. Similarly to the embodiment of the present invention that utilizes an artificial capsule bag 40, a translucent substance such as a liquid, gel, oil or sol, or any similar substance, is placed within capsular bag 26 to slow movement of the lens body 38. The structure and composition of the lens body 38 is also as described above with respect to FIGS. 2-4.

## Claims

1. A multifocal intraocular lens for insertion into an enucleated natural lens capsule of an eye, said lens comprising:
a lens body (38) having a substantially elliptical anterior surface (48), a substantially elliptical posterior surface (50), an upper portion (44), and a lower portion (46),
said lower portion having a semicircular shape in cross-section,
wherein said lens body encompasses the optical axis of the eye depending upon the position of the eye
**characterised in that**
the anterior surface and posterior surface of said lower portion taper upwardly to form upper portion to provide a tapering periphery (52) when viewed from the medial-lateral axis and
said anterior surface and said posterior surface of said upper portion each having at least one radius of curvature having its respective center positioned posteriorly the posterior surface of the lens body.

2. The multifocal intraocular lens of Claim 1, wherein said at least one radius of curvature of said posterior surface of said upper portion is shorter than that of said anterior surface of said upper portion.

3. The multifocal intraocular lens of Claim 1, wherein said anterior and posterior surfaces of said upper portion of said lens body each have multiple radii of curvature.

4. The multifocal intraocular lens of Claim 3, wherein said at least one multiple radii of curvature of said posterior surface of said lens body is shorter than said multiple radii of curvature of said anterior surface.

5. The multifocal intraocular lens of Claim 1, wherein the cross-sectional profile of said lens body is comma-shaped.

6. The multifocal intraocular lens of Claim 1, wherein said lens body has at least one index of refraction.

7. The multifocal intraocular lens of Claim 6, wherein said lens body has multiple indices of refraction.

8. The multifocal intraocular lens of Claim 7, wherein said lower portion of said lens body has a greater index of refraction than said upper portion.

9. The multifocal intraocular lens of Claim 1, wherein said lens body is substantially aspheric.

10. The multifocal intraocular lens of Claim 1, wherein said lens body comprises a material that is not colorless.

11. The multifocal intraocular lens of Claims 1 or 10, wherein said lens body comprises a material selected from the group consisting of silicone, acrylic, and polymethylmethacrylate.

12. A multifocal intraocular lens system for insertion of a multifocal intraocular lens into an artificial lens capsule for placement within an eye having a posterior chamber and an anterior chamber, said lens system comprising:
a lens body (38) having a substantially elliptical anterior surface (48);
said lens body having a substantially elliptical posterior surface (50);
said lens body having an upper portion (44) and a lower portion (46);
said lower portion having a semicircular shape;
an artificial lens capsule (40) having an anterior surface (54) and a posterior surface (60) and adapted to be positioned within the eye, said lens body being disposed within said artificial lens capsule;
a substance (62) dispersed within said artificial lens capsule for allowing said lens body to move within said artificial lens capsule; and
wherein said lens body encompasses the optical axis of the eye depending upon the position of the eye
**characterised in that**
the anterior surface and posterior surface of said lower portion taper upwardly to form upper portion to provide a tapering periphery (52) when viewed from the medial-lateral axis and
said anterior surface and said posterior surface of said upper portion each having at least one radius of curvature having its respective center positioned posteriorly the posterior surface of the lens body.

13. The multifocal intraocular lens system of Claim 12, wherein said artificial lens capsule is so dimensioned as to replace the natural lens capsule of the eye.

14. The multifocal intraocular lens system of Claim 12, wherein said artificial lens capsule is adapted to be positioned in the posterior chamber of an eye.

15. The multifocal intraocular lens system of Claim 12, wherein said artificial lens capsule is adapted to be positioned in the anterior chamber of an eye.

16. The multifocal intraocular lens system of Claim 12, wherein the distance between said anterior and posterior surface of said artificial lens capsule defines a thickness, said artificial lens capsule having a first axis extending generally perpendicular to said anterior and posterior surfaces and a second axis generally perpendicular to said first axis that defines a width.

17. The multifocal intraocular lens system of Claim 16, wherein the thickness of said artificial lens capsule along the first axis is smaller than its width along its second axis.

18. The multifocal intraocular lens system of Claim 16, wherein said artificial lens capsule is adapted to be positioned in the eye so that the first axis is approximately parallel with the optical axis of the eye.

19. The multifocal intraocular lens system of Claim 12, wherein said artificial lens capsule is not colorless.

20. The multifocal intraocular lens system of Claim 12, wherein said artificial lens capsule comprises a material selected from the group consisting of silicone, acrylic, and polymethylmethacrylate.

21. The multifocal intraocular lens system of Claim 12, wherein said artificial lens capsule is substantially pliable.

22. The multifocal intraocular lens system of Claim 12, wherein said artificial lens capsule has at least one index of refraction.

23. The multifocal intraocular lens system of Claim 12, wherein said at least one radius of curvature of the posterior surface of said upper portion of said lens body is shorter than that of said upper portion of said anterior surface of said lens body.

24. The multifocal intraocular lens system of Claim 12, wherein said anterior and posterior surfaces of said upper portion of said lens body each have multiple radii of curvature.

25. The multifocal intraocular lens system of Claim 24, wherein said at least one radii or curvature of said posterior surface of said lens body in the aggregate are shorter than said multiple radii of curvature of said anterior surface.

26. The multifocal intraocular lens system of Claim 12, wherein the cross-sectional shape of said lens body is comma-shaped.

27. The multifocal intraocular lens system of Claim 12, wherein said lower portion of said lens body has a greater index of refraction than said upper portion.

28. The multifocal intraocular lens system of Claim 12, wherein said lens body is substantially aspheric.

29. The multifocal intraocular lens system of Claim 12, wherein said lens body comprises a material that is not colorless.

30. The multifocal intraocular lens system of Claims 12 or 29, wherein said lens body comprises a material selected from the group consisting of silicone, acrylic, and polymethylmethacrylate.

31. The multifocal intraocular lens system of Claim 12, wherein said substance is not colorless.

32. The multifocal intraocular lens system of Claims 12 or 31, wherein said substance is a member of the group consisting of silicone, gel, sol, liquid, oil, and acrylic.

33. The multifocal intraocular lens system of Claim 12, wherein said substance slows movement of said lens body within said artificial lens capsule compared to movement of said lens body in the absence of said substance.

34. The multifocal intraocular lens system of Claim 12, wherein said lens system further comprises a securer.

35. The multifocal intraocular lens system of Claim 34, wherein said securer comprises at least two structures that extend from opposite sides of said lens capsule.

36. The multifocal intraocular lens system of Claim 34, wherein said structures are spring-like.

37. The multifocal intraocular lens system of Claim 35 or 36, wherein said structures comprise haptics or half-ticks..

38. The multifocal intraocular lens system of Claim 34,
wherein said securer holds said artificial lens capsule in place within the eye.

39. The multifocal intraocular lens of Claim 1, further wherein said lens body is immersed in a substance dispersed within said enucleated natural lens capsule for allowing said lens body to move within said enucleated natural lens capsule.

40. The multifocal intraocular lens of Claim 39, further wherein said substance is a member of the group consisting of silicone, gel, sol, liquid, oil, and acrylic.

41. The multifocal intraocular lens of Claim 39, further wherein said substance slows movement of said lens body within said enucleated natural lens capsule compared to movement of said lens body in the absence of said substance.

42. The multifocal intraocular lens of Claim 39, further wherein said substance is not colorless.

## Patentansprüche

1. Multifokale Intraokularlinse zum Einsetzen in eine enukleierte natürliche Linsenkapsel eines Auges, wobei die Linse umfasst:
einen Linsenkörper (38) mit einer im Wesentlichen elliptischen vorderen Fläche (48), einer im Wesentlichen elliptischen hinteren Fläche (50), einem oberen Abschnitt (44) und einem unteren Abschnitt (46),
wobei der untere Abschnitt im Querschnitt eine Halbkreisform hat,
wobei der Linsenkörper in Abhängigkeit von der Stellung des Auges die optische Achse des Auges umschließt,
**dadurch gekennzeichnet, dass**
die vordere Fläche und hintere Fläche des unteren Abschnitts sich nach oben hin verjüngen, um den oberen Abschnitt zu bilden, um, von der medianlateralen Achse aus gesehen, eine sich verjüngende Umrisslinie (52) bereitzustellen, und
wobei die vordere Fläche und die hintere Fläche des oberen Abschnitts jeweils mindestens einen Krümmungsradius haben, dessen jeweilige Mitte hinter der hinteren Fläche des Linsenkörpers angeordnet ist.

2. Multifokale Intraokularlinse nach Anspruch 1, wobei der mindestens eine Krümmungsradius der hinteren Fläche des oberen Abschnitts kürzer ist als derjenige der vorderen Fläche des oberen Abschnitts.

3. Multifokale Intraokularlinse nach Anspruch 1, wobei die vordere und hintere Fläche des oberen Abschnitts des Linsenkörpers jeweils mehrfache Krümmungsradien haben.

4. Multifokale Intraokularlinse nach Anspruch 3, wobei der mindestens eine mehrfache Krümmungsradius der hinteren Fläche des Linsenkörpers kürzer ist als die mehrfachen Krümmungsradien der vorderen Fläche.

5. Multifokale Intraokularlinse nach Anspruch 1, wobei das Querschnittsprofil des Linsenkörpers kommaförmig ist.

6. Multifokale Intraokularlinse nach Anspruch 1, wobei der Linsenkörper mindestens einen Brechungsindex hat.

7. Multifokale Intraokularlinse nach Anspruch 6, wobei der Linsenkörper mehrfache Brechungsindices hat.

8. Multifokale Intraokularlinse nach Anspruch 7, wobei der untere Abschnitt des Linsenkörpers einen größeren Brechungsindex hat als der obere Abschnitt.

9. Multifokale Intraokularlinse nach Anspruch 1, wobei der Linsenkörper im Wesentlichen asphärisch ist.

10. Multifokale Intraokularlinse nach Anspruch 1, wobei der Linsenkörper ein Material umfasst, das nicht farblos ist.

11. Multifokale Intraokularlinse nach Anspruch 1 oder 10, wobei der Linsenkörper ein Material umfasst, das aus der Gruppe ausgewählt ist, die aus Silikon, Acryl und Polymethylmethacrylat besteht.

12. Multifokafes Intraokularlinsensystem zum Einsetzen einer multifokalen Intraokularlinse in eine künstliche Linsenkapsel zum Einbringen in ein Auge mit einer hinteren Kammer und einer vorderen Kammer, wobei das Linsensystem umfasst:
einen Linsenkörper (38) mit einer im Wesentlichen elliptischen vorderen Fläche (48);
wobei der Linsenkörper eine im Wesentlichen elliptische hintere Fläche (50) hat;
wobei der Linsenkörper einen oberen Abschnitt (44) und einen unteren Abschnitt (46) hat;
wobei der untere Abschnitt eine Halbkreisform hat;
eine künstliche Linsenkapsel (40), die eine vordere Fläche (54) und eine hintere Fläche (60) hat und dazu angepasst ist, im Auge positioniert zu werden, wobei der Linsenkörper in der künstlichen Linsenkapsel angeordnet ist;
eine Substanz (62), die in der künstlichen Linsenkapsel dispergiert ist, um den Linsenkörper sich in der künstlichen Linsenkapsel bewegen zu lassen; und
wobei der Linsenkörper in Abhängigkeit von der Stellung des Auges die optische Achse des Auges umschließt,
**dadurch gekennzeichnet, dass**
die vordere Fläche und hintere Fläche des unteren Abschnitts sich nach oben hin verjüngen, um den oberen Abschnitt zu bilden, um, von der mediallateralen Achse aus gesehen, eine sich verjüngende Umrisslinie (52) bereitzustellen, und
wobei die vordere Fläche und die hintere Fläche des oberen Abschnitts jeweils mindestens einen Krümmungsradius haben, dessen jeweilige Mitte hinter der hinteren Fläche des Linsenkörpers angeordnet ist.

13. Multifokales Intraokularlinsensystem nach Anspruch 12, wobei die künstliche Linsenkapsel so dimensioniert ist, dass sie die natürliche Linsenkapsel des Auges ersetzt.

14. Multifokales Intraokularlinsensystem nach Anspruch 12, wobei die künstliche Linsenkapsel dazu angepasst ist, in der hinteren Kammer eines Auges positioniert zu werden.

15. Multifokales Intraokularlinsensystem nach Anspruch 12, wobei die künstliche Linsenkapsel dazu angepasst ist, in der vorderen Kammer eines Auges positioniert zu werden.

16. Multifokafes Intraokularlinsensystem nach Anspruch 12, wobei der Abstand zwischen der vorderen und hinteren Fläche der künstlichen Linsenkapsel eine Dicke definiert, wobei die künstliche Linsenkapsel eine erste Achse hat, die sich allgemein senkrecht zur vorderen und hinteren Fläche erstreckt, und eine zur ersten Achse allgemein senkrechte zweite Achse, die eine Breite definiert.

17. Multifokales Intraokularlinsensystem nach Anspruch 16, wobei die Dicke der künstlichen Linsenkapsel entlang der ersten Achse kleiner ist als ihre Breite entlang ihrer zweiten Achse.

18. Multifokales Intraokularlinsensystem nach Anspruch 16, wobei die künstliche Linsenkapsel dazu angepasst ist, so im Auge positioniert zu werden, dass die erste Achse annähernd parallel zur optischen Achse des Auges ist.

19. Multifokales Intraokularlinsensystem nach Anspruch 12, wobei die künstliche Linsenkapsel nicht farblos ist.

20. Multifokales Intraokularlinsensystem nach Anspruch 12, wobei die künstliche Linsenkapsel ein Material umfasst, das aus der Gruppe ausgewählt ist, die aus Silikon, Acryl und Polymethylmethacrylat besteht.

21. Multifokafes Intraokularlinsensystem nach Anspruch 12, wobei die künstliche Linsenkapsel im Wesentlichen nachgiebig ist.

22. Multifokales Intraokularlinsensystem nach Anspruch 12, wobei die künstliche Linsenkapsel mindestens einen Brechungsindex hat.

23. Multifokales Intraokularlinsensystem nach Anspruch 12, wobei der mindestens eine Krümmungsradius der hinteren Fläche des oberen Abschnitts des Linsenkörpers kürzer ist als derjenige des oberen Abschnitts der vorderen Fläche des Linsenkörpers.

24. Multifokales Intraokularlinsensystem nach Anspruch 12, wobei die vordere und hintere Fläche des oberen Abschnitts des Linsenkörpers jeweils mehrfache Krümmungsradien haben.

25. Multifokales Intraokularlinsensystem nach Anspruch 24, wobei die mindestens einen Krümmungsradien der hinteren Fläche des Linsenkörpers zusammengenommen kürzer sind als die mehrfachen Krümmungsradien der vorderen Fläche.

26. Multifokales Intraokularlinsensystem nach Anspruch 12, wobei die Querschnittsform des Linsenkörpers kommaförmig ist.

27. Multifokales Intraokularlinsensystem nach Anspruch 12, wobei der untere Abschnitt des Linsenkörpers einen höheren Brechungsindex hat als der obere Abschnitt.

28. Multifokales Intraokularlinsensystem nach Anspruch 12, wobei der Linsenkörper im Wesentlichen asphärisch ist.

29. Multifokales Intraokularlinsensystem nach Anspruch 12, wobei der Linsenkörper ein Material umfasst, das nicht farblos ist.

30. Multifokales Intraokularlinsensystem nach Anspruch 12 oder 29, wobei der Linsenkörper ein Material umfasst, das aus der Gruppe ausgewählt ist, die Silikon, Acryl und Polymethylmethacrylat umfasst.

31. Multifokales Intraokularlinsensystem nach Anspruch 12, wobei die Substanz nicht farblos ist.

32. Multifokales Intraokularlinsensystem nach Anspruch 12 oder 31, wobei die Substanz ein Mitglied der Gruppe ist, die aus Silikon, Gel, Sol, Flüssigkeit, Öl und Acryl besteht,

33. Multifokales Intraokularlinsensystem nach Anspruch 12, wobei die Substanz im Vergleich zu einer Bewegung des Linsenkörpers bei Nichtvorhandensein der Substanz die Bewegung des Linsenkörpers in der künstlichen Linsenkapsel verlangsamt.

34. Multifokales Intraokularlinsensystem nach Anspruch 12, wobei das Linsensystem darüber hinaus eine Fixierungseinrichtung umfasst.

35. Multifokales Intraokularlinsensystem nach Anspruch 34, wobei die Fixierungseinrichtung mindestens zwei Strukturen umfasst, die sich von entgegengesetzten Seiten der Linsenkapsel erstrecken.

36. Multifokales Intraokularlinsensystem nach Anspruch 34, wobei die Strukturen federartig sind.

37. Multifokafes Intraokularlinsensystem nach Anspruch 35 oder 36, wobei die Strukturen eine Haptik oder Häkchen umfassen.

38. Multifokales Intraokularlinsensystem nach Anspruch 34, wobei die Fixierungseinrichtung die künstliche Linsenkapsel im Auge an Ort und Stelle hält.

39. Multifokale Intraokularlinse nach Anspruch 1, wobei darüber hinaus der Linsenkörper in einer Substanz eingetaucht ist, die in der enukleierten natürlichen Linsenkapsel dispergiert ist, um den Linsenkörper sich in der enukleierten natürlichen Linsenkapsel bewegen zu lassen.

40. Multifokafe Intraokularlinse nach Anspruch 39, wobei darüber hinaus die Substanz ein Mitglied der Gruppe ist, die aus Silikon, Gel, Sol, Flüssigkeit, Öl und Acryl besteht.

41. Multifokale Intraokularlinse nach Anspruch 39, wobei darüber hinaus die Substanz im Vergleich zu einer Bewegung des Linsenkörpers bei Nichtvorhandensein der Substanz die Bewegung des Linsenkörpers in der enukleierten natürlichen Linsenkapsel verlangsamt.

42. Muftifokale Intraokularlinse nach Anspruch 39, wobei darüber hinaus die Substanz nicht farblos ist.

## Revendications

1. Lentille intraoculaire multifocale pour insertion dans une capsule de cristallin naturel énucléé d'un oeil, ladite lentille comprenant :
un corps de lentille (38) ayant une surface antérieure sensiblement elliptique (48), une surface postérieure sensiblement elliptique (50), une portion supérieure (44) et une portion inférieure (46),
ladite portion inférieure ayant une forme semicirculaire en coupe transversale,
dans laquelle ledit corps de lentille englobe l'axe optique de l'oeil en fonction de la position de l'oeil
**caractérisée en ce que**
la surface antérieure et la surface postérieure de ladite portion inférieure s'amincissent vers le haut pour former une portion supérieure pour donner une périphérie amincie (52) lorsque vue depuis l'axe médio-latéral et
ladite surface antérieure et ladite surface postérieure de ladite portion supérieure ont chacune au moins un rayon de courbure ayant son centre respectif positionné postérieurement à la surface postérieure du corps de lentille.

2. Lentille intraoculaire multifocale selon la revendication 1, dans laquelle ledit au moins un rayon de courbure de ladite surface postérieure de ladite portion supérieure est plus court que celui de ladite surface antérieure de ladite portion supérieure.

3. Lentille intraoculaire multifocale selon la revendication 1, dans laquelle lesdites surfaces antérieure et postérieure de ladite portion supérieure dudit corps de lentille ont chacune plusieurs rayons de courbure.

4. Lentille intraoculaire multifocale selon la revendication 3, dans laquelle lesdits au moins un plusieurs rayons de courbure de ladite surface postérieure dudit corps de lentille sont plus courts que lesdits plusieurs rayons de courbure de ladite surface antérieure.

5. Lentille intraoculaire multifocale selon la revendication 1, dans laquelle le profil de section transversale dudit corps de lentille est en forme de virgule.

6. Lentille intraoculaire multifocale selon la revendication 1, dans laquelle ledit corps de lentille a au moins un indice de réfraction.

7. Lentille intraoculaire multifocale selon la revendication 6, dans laquelle ledit corps de lentille a plusieurs indices de réfraction.

8. Lentille intraoculaire multifocale selon la revendication 7, dans laquelle ladite portion inférieure dudit corps de lentille a un indice de réfraction supérieur à ladite portion supérieure.

9. Lentille intraoculaire multifocale selon la revendication 1, dans laquelle ledit corps de lentille est sensiblement asphérique.

10. Lentille intraoculaire multifocale selon la revendication 1, dans laquelle ledit corps de lentille comprend un matériau qui n'est pas incolore.

11. Lentille intraoculaire multifocale selon les revendications 1 ou 10, dans laquelle ledit corps de lentille comprend un matériau choisi parmi le groupe constitué de silicone, d'acrylique et de polyméthacrylate de méthyle.

12. Système de lentille intraoculaire multifocale pour insertion d'une lentille intraoculaire multifocale dans une capsule de cristallin artificiel en vue d'un placement à l'intérieur d'un oeil ayant une chambre postérieure et une chambre antérieure, ledit système de lentille comprenant :
un corps de lentille (38) ayant une surface antérieure sensiblement elliptique (48) ;
ledit corps de lentille ayant une surface postérieure sensiblement elliptique (50) ;
ledit corps de lentille ayant une portion supérieure (44) et une portion inférieure (46) ;
ladite portion inférieure ayant une forme semicirculaire ;
une capsule de cristallin artificiel (40) ayant une surface antérieure (54) et une surface postérieure (60) et adaptée pour être positionnée à l'intérieur de l'oeil, ledit corps de lentille étant disposé à l'intérieur de ladite capsule de cristallin artificiel ;
une substance (62) dispersée à l'intérieur de ladite capsule de cristallin artificiel pour permettre audit corps de lentille de se déplacer à l'intérieur de ladite capsule de cristallin artificiel ; et
dans lequel ledit corps de lentille englobe l'axe optique de l'oeil en fonction de la position de l'oeil
**caractérisé en ce que**
la surface antérieure et la surface postérieure de ladite portion inférieure s'amincissent vers le haut pour former une portion supérieure pour donner une périphérie amincie (52) lorsque vue depuis l'axe médio-latéral et
ladite surface antérieure et ladite surface postérieure de ladite portion supérieure ont chacune au moins un rayon de courbure ayant son centre respectif positionné postérieurement à la surface postérieure du corps de lentille.

13. Système de lentille intraoculaire multifocale selon la revendication 12, dans lequel ladite capsule de cristallin artificiel est dimensionnée de manière à remplacer la capsule de cristallin naturel de l'oeil.

14. Système de lentille intraoculaire multifocale selon la revendication 12, dans lequel ladite capsule de cristallin artificiel est adaptée pour être positionnée dans la chambre postérieure d'un oeil.

15. Système de lentille intraoculaire multifocale selon la revendication 12, dans lequel ladite capsule de cristallin artificiel est adaptée pour être positionnée dans la chambre antérieure d'un oeil.

16. Système de lentille intraoculaire multifocale selon la revendication 12, dans lequel la distance entre lesdites surfaces antérieure et postérieure de ladite capsule de cristallin artificiel définit une épaisseur, ladite capsule de cristallin artificiel ayant un premier axe s'étendant généralement perpendiculaire auxdites surfaces antérieure et postérieure et un second axe généralement perpendiculaire audit premier axe qui définit une largeur.

17. Système de lentille intraoculaire multifocale selon la revendication 16, dans lequel l'épaisseur de ladite capsule de cristallin artificiel le long du premier axe est plus petite que sa largeur le long de son second axe.

18. Système de lentille intraoculaire multifocale selon la revendication 16, dans lequel ladite capsule de cristallin artificiel est adaptée pour être positionnée dans l'oeil de sorte que le premier axe est approximativement parallèle à l'axe optique de l'oeil.

19. Système de lentille intraoculaire multifocale selon la revendication 12, dans lequel ladite capsule de cristallin artificiel n'est pas incolore.

20. Système de lentille intraoculaire multifocale selon la revendication 12, dans lequel ladite capsule de cristallin artificiel comprend un matériau choisi parmi le groupe constitué de silicone, d'acrylique et de polyméthacrylate de méthyle.

21. Système de lentille intraoculaire multifocale selon la revendication 12, dans lequel ladite capsule de cristallin artificiel est sensiblement souple.

22. Système de lentille intraoculaire multifocale selon la revendication 12, dans lequel ladite capsule de cristallin artificiel a au moins un indice de réfraction.

23. Système de lentille intraoculaire multifocale selon la revendication 12, dans lequel ledit au moins un rayon de courbure de la surface postérieure de ladite portion supérieure dudit corps de lentille est plus court que celui de ladite portion supérieure de ladite surface antérieure dudit corps de lentille.

24. Système de lentille intraoculaire multifocale selon la revendication 12, dans lequel lesdites surfaces antérieure et postérieure de ladite portion supérieure dudit corps de lentille ont chacune plusieurs rayons de courbure.

25. Système de lentille intraoculaire multifocale selon la revendication 24, dans lequel lesdits au moins un rayon de courbure de ladite surface postérieure dudit corps de lentille sont dans l'ensemble plus courts que lesdits plusieurs rayons de courbure de ladite surface antérieure.

26. Système de lentille intraoculaire multifocale selon la revendication 12, dans lequel la forme de section transversale dudit corps de lentille est en forme de virgule.

27. Système de lentille intraoculaire multifocale selon la revendication 12, dans lequel ladite portion inférieure dudit corps de lentille a un indice de réfraction plus grand que ladite portion supérieure.

28. Système de lentille intraoculaire multifocale selon la revendication 12, dans lequel ledit corps de lentille est sensiblement asphérique.

29. Système de lentille intraoculaire multifocale selon la revendication 12, dans lequel ledit corps de lentille comprend un matériau qui n'est pas incolore.

30. Système de lentille intraoculaire multifocale selon les revendications 12 ou 29, dans lequel ledit corps de lentille comprend un matériau choisi parmi le groupe constitué de silicone, d'acrylique et de polyméthacrylate de méthyle.

31. Système de lentille intraoculaire multifocale selon la revendication 12, dans lequel ladite substance n'est pas incolore.

32. Système de lentille intraoculaire multifocale selon les revendications 12 ou 31, dans lequel ladite substance est un élément du groupe constitué de silicone, de gel, de sol, de liquide, d'huile et d'acrylique.

33. Système de lentille intraoculaire multifocale selon la revendication 12, dans lequel ladite substance ralentit un mouvement dudit corps de lentille à l'intérieur de ladite capsule de cristallin artificiel comparé à un mouvement dudit corps de lentille en l'absence de ladite substance.

34. Système de lentille intraoculaire multifocale selon la revendication 12, dans lequel ledit système de lentille comprend en outre un élément de fixation.

35. Système de lentille intraoculaire multifocale selon la revendication 34, dans lequel ledit élément de fixation comprend au moins deux structures qui s'étendent à partir de côtés opposés de ladite capsule de lentille.

36. Système de lentille intraoculaire multifocale selon la revendication 34, dans lequel lesdites structures sont analogues à un ressort.

37. Système de lentille intraoculaire multifocale selon la revendication 35 ou 36, dans lequel lesdites structures comprennent des haptiques ou des demies coches.

38. Système de lentille intraoculaire multifocale selon la revendication 34, dans lequel ledit élément de fixation maintient ladite capsule de cristallin artificiel en place à l'intérieur de l'oeil.

39. Lentille intraoculaire multifocale selon la revendication 1, également dans laquelle ledit corps de lentille est immergé dans une substance dispersée à l'intérieur de ladite capsule de cristallin naturel énucléé pour permettre audit corps de lentille de se déplacer à l'intérieur de ladite capsule de cristallin naturel énucléé.

40. Lentille intraoculaire multifocale selon la revendication 39, également dans laquelle ladite substance est un élément du groupe constitué de silicone, de gel, de sol, de liquide, d'huile et d'acrylique.

41. Lentille intraoculaire multifocale selon la revendication 39, également dans laquelle ladite substance ralentit un mouvement dudit corps de lentille à l'intérieur de ladite capsule de cristallin naturel énucléé comparé à un mouvement dudit corps de lentille en l'absence de ladite substance.

42. Lentille intraoculaire multifocale selon la revendication 39, également dans laquelle ladite substance n'est pas incolore.
